# EUROPEAN PATENT APPLICATION

(11) **EP 1 873 242 A2**
(43) Date of publication of application: **02.01.2008**
(21) Application number: 07252564.5
(22) Date of filing: 25.06.2007
(51) Int. Cl.: C12N 15/10

(54) **Filter device for the isolation of a nucleic acid**

(30) Priority: 29.06.2006 US 817504 P; 18.01.2007 US 881058 P
(71) Applicant: MILLIPORE CORPORATION, Billerica Massachusetts 01821 (US)
(72) Inventor: Baggio, Ricky Francis, Waltham, MA 02453 (US); Gagne, George A. Jr., Dracut, MA 01826 (US); Aysola, Manjula, c/o Millipore Corporation, Billerica, MA 01821 (US); Murrell, Julie R., c/o Millipore Corporation, Billerica, MA 01821 (US)
(74) Representative: Kirkham, Nicholas Andrew

(57) **Abstract**

The invention relates to methods of isolating nucleic acids from a sample using a filter device comprising a plurality of membranes. The invention also provides for devices comprising a plurality of membranes and kits suitable for isolating nucleic acids from a sample. The invention also provides for nucleic acids which bind to at least a portion of a microorganism genome and methods of using the same.

SEQUENCE LISTING

<110>  MILLIPORE CORPORATION

<120>  FILTER DEVICE FOR THE ISOLATION OF A NUCLEIC ACID

<130>  P9769EP00

<160>  12

<170>  PatentIn version 3.4

<210>  1
<211>  18
<212>  DNA
<213>  ARTIFICIAL

<220>
<223>  ARTIFICIAL PRIMER

<400>  1
cacacggcgg gtgctaac                                                    18

<210>  2
<211>  22
<212>  DNA
<213>  ARTIFICIAL

<220>
<223>  ARTIFICIAL PRIMER

<400>  2
ccacaacttt gggaccttag ct                                               22

<210>  3
<211>  25
<212>  DNA
<213>  ARTIFICIAL

<220>
<223>  ARTIFICIAL PRIMER

<400>  3
ccgtcgtgaa aagggaaaca accca                                            25

<210>  4
<211>  22
<212>  DNA
<213>  ARTIFICIAL

<220>
<223>  ARTIFICIAL PRIMER

<400>  4
gcaaacagca tggtgaaaat tg                                               22

<210>  5

<211>  23
<212>  DNA
<213>  ARTIFICIAL

<220>
<223>  ARTIFICIAL PRIMER

<400>  5
cctgaaccaa agcttgtttc atc                                              23


<210>  6
<211>  24
<212>  DNA
<213>  ARTIFICIAL

<220>
<223>  ARTIFICIAL PROBE

<400>  6
tggaccagca ccagagcgct acac                                             24


<210>  7
<211>  24
<212>  DNA
<213>  ARTIFICIAL

<220>
<223>  ARTIFICIAL PRIMER

<400>  7
aaatgatccg cctgagtagt atgc                                             24


<210>  8
<211>  19
<212>  DNA
<213>  ARTIFICIAL

<220>
<223>  ARTIFICIAL PRIMER

<400>  8
catgctccac cgcttgtgc                                                   19


<210>  9
<211>  30
<212>  DNA
<213>  ARTIFICIAL

<220>
<223>  ARTIFICIAL PROBE

<400>  9
cgcaagagtg aaacttaaag gaattgacgg                                       30


<210>  10
<211>  24

<212>  DNA
<213>  ARTIFICIAL

<220>
<223>  ARTIFICIAL PRIMER

<400>  10
aaacatcccg cctgggtagt acat                                              24


<210>  11
<211>  21
<212>  DNA
<213>  ARTIFICIAL

<220>
<223>  ARTIFICIAL PRIMER

<400>  11
caacatgctc caccacttgt g                                                 21


<210>  12
<211>  30
<212>  DNA
<213>  ARTIFICIAL

<220>
<223>  ARTIFICIAL PROBE

<400>  12
cgcaagaatg aaacttaaac ggaattgacg                                        30

## Description

This application claims the benefit of U.S. Provisional Application No. 60/817,504 filed June 29, 2006 and U.S. Provisional Application No. 60/881,058 filed January 18, 2007 both of which are filed herewith and are hereby incorporated by reference in their entirety.

### Field of the Invention

The invention relates generally to the field of molecular biology. In certain specific embodiments the invention provides devices, kits and methods relating to the isolation and detection of nucleic acids.

### Background of the Invention

Isolating nucleic acids is typically the first step of most molecular biological inquiries including PCR, gene cloning, sequencing, Southern analysis, Northern analysis, nuclease protection assays, RT-PCR, RNA mapping, in vitro translation, in vitro transcription, including transcription/amplification reactions and cDNA library construction. Obtaining high quality intact nucleic acids suitable for analysis is thus often a useful and desirable starting point for subsequent analyses. While a variety of techniques for isolating nucleic acids from a sample have been described (see, e.g., U.S. Patent Nos.5,075,430; 5,234,809; 5,155,018; 6,274,308; 6,277,648; 6,958,392; 6,953,686; 6,310,199; 6,992,182; 6,475,388; 5,075,430; U.S. Patent Publication No. 20060024701; European Patent No. EP0765335; Boom et al. 1990, J. Clinical Microbiology 28:495), it would be, nonetheless, beneficial to provide a method of isolating a nucleic acid from a sample that was fast, economical, and produced high yields. It would also be useful if the method minimized the required manipulation of the sample, permitted the use of primarily liquid handling steps and could be performed using a single device, e.g. a filtering device. Certain embodiments of the invention described herein provide for such a method. Other embodiments of the invention described herein provide for devices and kits which may be used for isolating nucleic acids from a sample. Still other embodiments of the invention provide for the detection of a nucleic acid in a sample.

### SUMMARY OF THE INVENTION

In certain embodiments the invention provides a method of isolating a nucleic acid from a sample comprising a) contacting a filter device comprising a plurality of porous membranes with the sample such that the sample contacts the plurality of porous membranes sequentially beginning with a first porous membrane and followed by at least a second porous membrane, b) lysing the sample on the first porous membrane, wherein the first porous membrane has a nominal pore size which is smaller than the nominal pore size of the second porous membrane c) applying an external force to the filter device, thereby isolating the nucleic acid on at least one of the plurality of porous membranes. The sample may be a cellular sample or a viral sample e.g. comprised of viral particles. Optionally, the isolated nucleic acid may be eluted from one of the porous membranes.

In other embodiments the invention provides a method of isolating a nucleic acid from a sample comprising contacting a filter device comprising a plurality of porous membranes with the sample such that the sample contacts the plurality of porous membranes sequentially beginning with a first porous membrane comprising a polymer and followed by at least a second porous membrane comprising silicate thereby isolating the nucleic acid on at least one of the plurality of porous membranes. The polymer membrane may have a smaller nominal pore size compared to the silicate membrane. The sample may be a cellular sample or a viral sample either or both of which may be lysed in situ on one of the membranes, e.g. the first porous membrane. Optionally, the isolated nucleic acid may be eluted from one of the porous membranes.

In yet other embodiments the invention provides a method of isolating a nucleic acid from a sample comprising contacting a filter device comprising a plurality of porous membranes with the sample such that the sample contacts the plurality of porous membranes sequentially beginning with a first porous membrane comprising a polysaccharide and followed by at least a second porous membrane comprising silicate thereby isolating the nucleic acid on at least one of the plurality of porous membranes. The polysaccharide membrane may have a smaller nominal pore size compared to the silicate membrane. The sample may be a cellular sample or a viral sample either or both of which may be lysed in situ on one of the membranes, e.g. the first porous membrane. Optionally, the isolated nucleic acid may be eluted from one of the porous membranes.

In still other embodiments the invention provides a method of isolating a nucleic acid from a sample comprising contacting a filter device comprising a plurality of porous membranes with the sample such that the sample contacts the plurality of porous membranes sequentially beginning with a first porous membrane comprising a polymer comprised of a sulfonyl group and followed by at least a second porous membrane comprising silicate thereby isolating the nucleic acid on at least one of the plurality of porous membranes. The polymer membrane comprised of a sulfonyl group may have a smaller nominal pore size compared to the silicate membrane. The sample may be a cellular sample or a viral sample either or both of which may be lysed in situ on one of the membranes, e.g. the first porous membrane. Optionally, the isolated nucleic acid may be eluted from one of the porous membranes.

In yet other embodiments the invention provides a method of isolating a nucleic acid from a sample comprising contacting a filter device comprising a plurality of porous membranes with the sample such that the sample contacts the plurality of porous membranes sequentially beginning with a first porous membrane comprising a polymer comprised of a sulfone group and followed by at least a second porous membrane comprising a polysaccharide thereby isolating the nucleic acid on at least one of the plurality of porous membranes. The polymer membrane comprised of a sulfonyl group may have a smaller nominal pore size compared to the polysaccharide membrane. The sample may be a cellular sample or a viral sample either or both of which may be lysed in situ on one of the membranes, e.g. the first porous membrane. Optionally, the isolated nucleic acid may be eluted from one of the porous membranes.

In some embodiments the invention provides a method of isolating RNA from a sample comprising contacting a filter device comprising a plurality of porous membranes with the sample such that the sample first contacts a membrane comprising mixed cellulose ester followed by a membrane comprising a glass fiber, wherein the nominal pore size of the mixed cellulose ester membrane is smaller than the nominal pore size of the glass fiber membrane and optionally eluting the RNA from the device thereby isolating RNA from the sample. The sample may be a cellular sample or a viral sample either or both of which may be lysed in situ on one of the membranes, e.g. the first porous membrane.

In some embodiments the invention provides a method of isolating DNA from a sample comprising contacting a filter device comprising a plurality of porous membranes with the sample such that the sample first contacts a membrane comprising polymer comprised of a sulfonyl group followed by a membrane comprising a glass fiber, wherein the nominal pore size of the polymer membrane comprised of a sulfonyl group is smaller than the nominal pore size of the glass fiber membrane and eluting the DNA from the device thereby isolating DNA from the sample. In certain embodiments at least some of the DNA is isolated on the polymer membrane comprised of the sulfonyl group. In other embodiments at least some of the DNA is isolated on the glass fiber membrane. The sample may be a cellular sample or a viral sample either or both of which may be lysed in situ on one of the membranes, e.g. the first porous membrane. Optionally, the isolated DNA may be eluted from one of the porous membranes.

In yet other embodiments the invention provides a method of isolating RNA from a cellular sample comprising a) contacting the cellular sample with a first membrane comprising a polymer; b) applying an external force to the first membrane such that the cellular sample is retained on a surface of the first membrane; c) contacting the cellular sample retained on the surface of the first membrane with a lysis buffer, such that the cells in the sample lyse, and an RNA capture buffer, such that the RNA in the sample elutes from the first membrane and contacts a second membrane comprised of silica; d) applying an external force to the first and second membranes such that the lysis buffer and RNA capture buffer flow through the first and second membranes of c); e) optionally contacting the first and/or second membranes of d) with one or more wash buffers; f) optionally applying an external force to the first and/or second membrane of e) such that the wash buffers flow through the first and/or second membranes; g) adding RNase free water such that at least some of the RNA elutes from the silica membrane thereby isolating RNA from the sample. In some embodiments the lysis buffer may be the same buffer as the RNA capture buffer such that the cells comprising the sample are lysed.

In still other embodiments the invention provides a method of isolating DNA from a sample comprising a) contacting the sample with a plurality of membranes, wherein the first membrane is comprised of a polymer, and at least one additional membrane, which contacts the sample subsequent to the first polymer membrane, wherein the at least one additional membrane is comprised of silica; b) applying an external force to the polymer membrane such that the sample is retained on a surface of the polymer membrane; c) contacting the sample retained on the surface of the polymer membrane with a lysis buffer, such that the sample containing the DNA lyses; d) applying an external force to at least one of the first and second membranes such that the lysis buffer flow through at least the first and second membranes of c); e) optionally contacting the first and second membranes of d) with one or more wash buffers; f) optionally applying an external force to at least the second membrane of e) such that the wash buffers flow through at least the second membrane; g) adding nuclease free water to the plurality of membranes such that at least some of the DNA elutes from the at least one membrane thereby isolating DNA from the sample.

In certain other embodiments the invention provides a filter device suitable for isolating nucleic acids from a sample comprising a plurality of porous membranes arranged sequentially such that a sample applied to the filter device contacts a first porous membrane followed by at least a second porous membrane, wherein the first porous membrane has a nominal pore size which is smaller than the nominal pore size of the second porous membrane.

In further embodiments the invention provides a filter device suitable for isolating nucleic acids from a sample comprising a plurality of porous membranes arranged sequentially such that a sample applied to the filter device contacts a first porous membrane comprising a polymer followed by at least a second porous membrane comprising silica. The polymer membrane may have a smaller nominal pore size compared to the silicate membrane.

In still other embodiments the invention provides a filter device suitable for isolating nucleic acids from a sample comprising a plurality of porous membranes arranged sequentially such that a sample applied to the filter device contacts a first porous membrane comprising a polymer comprised of a sulfonyl group followed by at least a second porous membrane comprising silica. The polymer membrane comprised of a sulfonyl group may have a smaller nominal pore size compared to the silicate membrane.

In yet further embodiments the invention provides a filter device suitable for isolating nucleic acids from a sample comprising a plurality of porous membranes arranged sequentially such that a sample applied to the filter device contacts a first porous membrane comprising a polysacharride followed by at least a second porous membrane comprising silica. The polysaccharide membrane may have a smaller nominal pore size compared to the silicate membrane.
In still other embodiments the invention provides a filter device suitable for isolating nucleic acids from a sample comprising a plurality of porous membranes arranged sequentially such that a sample applied to the filter device contacts a first porous membrane comprising a polymer comprised of a sulfonyl group followed by at least a second porous membrane comprising a polysacharride. The polymer membrane comprised of a sulfonyl group may have a smaller nominal pore size compared to the polysaccharide membrane.

In still other embodiments the invention provides a filter device suitable for isolating RNA from a sample comprising a plurality of membranes arranged sequentially such that a sample applied to the filter device contacts a first porous membrane comprising mixed cellulose acetate followed by at least a second porous membrane comprising glass fiber, wherein the first porous membrane may have a nominal pore size which is smaller than the nominal pore size of the second porous membrane.

In still further embodiments the invention provides a filter device suitable for isolating DNA from a sample comprising a plurality of membranes arranged sequentially such that a sample applied to the filter device contacts a first porous membrane comprising polymer comprised of a sulfonyl group followed by at least a second porous membrane comprising glass fiber, wherein the first porous membrane has a nominal pore size which may be smaller than the nominal pore size of the second porous membrane.

In still other embodiments the invention provides a filter device suitable for isolating DNA from a sample comprising a plurality of membranes arranged sequentially such that a sample applied to the filter device contacts a first porous membrane comprising polymer comprised of a sulfonyl group, followed by a second porous membrane comprising polymer comprised of a sulfonyl group, followed by at least a third porous membrane comprising glass fiber, wherein the first porous membrane has a nominal pore size which is smaller than the nominal pore size of the glass fiber membrane.

In yet other embodiments the invention provides a kit for isolating a nucleic acid from a sample comprising a) a filter device suitable for isolating nucleic acids from a sample comprising a plurality of porous membranes arranged sequentially such that a sample applied to the filter device contacts a first porous membrane followed by at least a second porous membrane, wherein the first porous membrane may have a nominal pore size which is smaller than the nominal pore size of the second porous membrane; and b) at least one container.

In still other embodiments the invention provides a kit for isolating a nucleic acid from a sample comprising a) a filter device suitable for isolating nucleic acids from a sample comprising a plurality of porous membranes arranged sequentially such that a sample applied to the filter device contacts a first porous membrane comprising a polymer followed by at least a second porous membrane comprising silica; and b) at least one container. The polymer membrane may have a smaller nominal pore size compared to the silicate membrane.

In yet other embodiments the invention provides a kit for isolating a nucleic acid from a sample comprising a) a filter device suitable for isolating nucleic acids from a sample comprising a plurality of porous membranes arranged sequentially such that a sample applied to the filter device contacts a first porous membrane comprising a polysaccharide followed by at least a second porous membrane comprising a silicate; and b) at least one container. The polysaccharide membrane may have a smaller nominal pore size compared to the silicate membrane.

In further embodiments the invention provides a kit for isolating a nucleic acid from a sample comprising a) a filter device suitable for isolating nucleic acids from a sample comprising a plurality of porous membranes arranged sequentially such that a sample applied to the filter device contacts a first porous membrane comprising a polymer comprised of a sulfonyl group followed by at least a second porous membrane comprising a silicate; and b) at least one container. The polymer membrane comprised of a sulfonyl group may have a smaller nominal pore size compared to the silicate membrane.

In still other embodiments the invention provides a kit for isolating a nucleic acid from a sample comprising a) a filter device suitable for isolating nucleic acids from a sample comprising a plurality of porous membranes arranged sequentially such that a sample applied to the filter device contacts a first porous membrane comprising a polymer comprised of a sulfonyl group followed by at least a second porous membrane comprising a polysaccharide; and b) at least one container. The polymer membrane comprised of a sulfonyl group may have a smaller nominal pore size compared to the polysaccharide membrane.

In yet further embodiments the invention provides an automated system for isolating a nucleic acid from a sample comprising a) a filter device suitable for isolating a nucleic acid from a sample; b) a pump, c) a program logic controller(PLC), d) at least one container suitable for holding a sample, e) optionally one or more containers suitable for holding one or more reagents or buffers, f) optionally one or more receptacles suitable for receiving a filtrate from the filter device, an eluate from a filter device and/or a waste solution from a filter device; g) a plurality of pinch valves; h) tubing and i) optionally a syringe cartridge which feeds one or more fluids from the tubing into the filter device.

In further embodiments the invention provides one or more oligonucleotides suitable for detecting a microorganism in sample. The microorganism may include a mycoplasma, a virus and the like.

In still further embodiments the invention provides a method of detecting a microorganism in a sample comprising a) contacting the sample with one or more oligonucleotides which specifically bind to a target nucleic acid comprising at least a portion of the genome of the microorganism b) amplifying the target nucleic acid thereby detecting the microorganism in the sample. Optionally, the target nucleic acid may be contacted with an oligonucleotide probe which specifically binds to at least a portion of the target nucleic acid thereby detecting the microorganism in the sample.

### BRIEF DESCRIPTION OF THE DRAWINGS

Figures 1 a and 1 b show one example of a filter device of the invention which is comprised of a micropartition device.
   Figure 2 shows an example of a filter device of the invention.
   Figures 3a and 3b show an example of a filter device of the invention in multiwell format suitable for receiving multiple samples or large volume samples.
   Figure 4 shows another example of a filter device of the invention in multiwell format suitable for receiving multiple samples or large volume samples.
   Figure 5 shows a single sample version of a filter device of the invention.
   Figure 6a shows a closed version of a filter device of the invention: vented and non-vented.
   Figure 6b shows a stacked version of a filter device of the invention.
   Figure 7 shows a controllable, automated system with a closed, vented version of the filter device of the invention.
   Figure 8 is a photograph of an agarose gel showing rRNA isolated according to the methods of the invention compared to rRNA isolated using a RNAeasy® column (Qiagen, Valencia, CA).
   Figure 9 is a graph showing relative purification of RNA from samples.

### DESCRIPTION OF THE EMBODIMENTS

### Methods of Isolating Nucleic Acids

In some embodiments the invention provides a method of isolating a target nucleic acid from a sample which is fast, easy to perform and requires a minimal number of manipulations to the sample. The method offers the advantage of accepting crude cellular input, e.g., prokaryotic or eukaryotic cells, and discharging a selective biomolecule output. The crude cell input may merely involving lysing a cellular sample, e.g. in situ on the surface of one of a plurality of membranes comprising a filtration device. The lysate may be processed so as to isolate a nucleic acid according to the methods described herein without having to clarify the lysate for example by centrifugation. The lysate thus may comprise the target nucleic acid along with cellular debris, growth media and growth media additives.

In specific embodiments the invention provides a method of isolating RNA on a glass fiber membrane wherein the glass fiber membrane is part of a filtration device comprising a plurality of membranes. In other embodiments the invention provides a method of isolating DNA on a polymer membrane such as a polymer membrane comprised of a sulfonyl group wherein the polymer membrane is part of a filtration device comprising a plurality of membranes.

Where the target nucleic acid is RNA, the invention provides a method of isolating RNA that does not require the use of DNAse, or alternatively, requires the use of less DNAse than is required by previously described methods, e.g., Qiagen columns (Qiagen, Inc., Valencia, CA) thereby saving time and reagent cost. Typically, the nucleic acid will be isolated on a porous membrane in a denatured state thus facilitating further manipulation, e.g. PCR including RT-PCR, transcription mediated amplification (TMA). Isolation of the target nucleic acid may be used for many down stream analytical applications including, for example, identification of a pathogenic or non-pathogenic organism of interest, such as a bacterium.

In certain embodiments the invention provides a method of isolating nucleic acid from a sample comprised of whole cells such as prokaryotic or eukaryotic cells. The cells may be contacted with a first membrane, lysed and the target nucleic acid may be isolated on a second or subsequent membrane. Alternatively the target nucleic acid may be isolated on both the first and second membrane. The sample may be applied to a filter device comprised of a plurality of membranes such that the sample contacts each of the plurality of membranes sequentially. Thus, the sample may contact a first membrane followed by at least a second membrane. In some embodiments the sample may contact three or more membranes.

After the sample contacts the first membrane of the device an external force may be applied to the device such that the sample (e.g. the target nucleic acid or at least a portion of the target nucleic acid) is retained on the surface of the first membrane, but the solution, or liquid, e.g. culture media, containing the sample flows through the filter device. In some embodiments the force may be provided by gravity or by a vacuum attached to the filter device, e.g. via an outlet on the bottom of the device. When the external force is a vacuum, the vacuum may provide a pressure difference of 20-800 mbars. In other embodiments the force may be provide by positive pressure which may be applied from the top of the filtration device, e.g, by the action of a piston or member, where the device is comprised of a cylinder, or the force of a syringe. In still other embodiments the force may be a centrifugal force applied by placing the device in a centrifuge. Centrifugal forces ranging from 1Xg -15,000Xg may be used. In still other embodiments the solvent may be removed by suction, e.g. from above surface of the first membrane, or simply decanted after allowing the sample to settle.

The method may comprise contacting the sample with various buffers after the sample contacts the first of a plurality of porous membrane comprising the filter device. Thus after the sample contacts the first membrane comprising a filter device, and optionally a force as described above has been applied to the filter device, it may be contacted with a first buffer. Where the sample is comprised of cells and/or viral particles, the first buffer may be a lysis buffer. Any lysis buffer known in the art may be used. Typically, the lysis buffer will comprise a buffering salt, a detergent and an enzyme, e.g. a protease. The enzyme may be for example proteinase K or lysozyme. In one embodiment the lysis buffer may be comprised of 50mM Tris-HCl pH8.0; 100mM EDTA; 2% Triton X-100.

After lysis the sample may be contacted with a precipitating reagent such that at least a portion of the nucleic acid contained in the sample precipitates onto a surface of the first porous membrane. The precipitating reagent may be a solution comprising a guanidine salt, (e.g. guanidine-HCl, guanidine thiocyanate), and an alcohol, e.g. ethanol. In some embodiments the precipitating reagent comprises a final alcohol concentration ranging from 25-50%. The sample may be incubated with a precipitating reagent long enough for at least some of the nucleic acid to precipitate onto the surface of the first membrane. In some embodiments the incubation time ranges from 1-10 minutes, 1-5 minutes, 30 seconds to 3 minutes, 30 seconds to 20 minutes, 30 seconds to an hour or an hour or more. The precipitating reagent and the lysis buffer may be removed from the sample using any of the techniques described above for the removal of the solvent from the sample, e.g. application of an external force.

Where the target analyte is RNA an RNA capture buffer may be added which permits the target nucleic acid to pass through the first membrane and adsorb to or precipitate onto the second membrane such that the target nucleic acid is isolated from the sample. Examples of suitable RNA capture buffers include 4M guanidine hydrochloride; 50mM Tris-HCl pH8.0; 100mM EDTA; 2% Triton X-100; 50% ethanol. One percent sodium dodecyl sulfate may be used as a substitute for the 2% Triton X-100. Another RNA capture buffer may be comprised of 4M guanidine hydrochloride; 50mM Tris-HCl pH8.0; 100mM EDTA; 5% ethyl acetate; 50% ethanol.

In between any of the steps, including a) contacting the sample with the filter device, b) precipitation, c) elution of the target, one or more wash buffers may be applied to the sample retained on the device. For example, after removal of the precipitating reagent and the lysis buffer, the sample may be contacted with a wash buffer to remove unwanted material resulting from lysis and enzymatic degradation. Any wash buffer known in the art may be used, e.g. phosphate buffered saline (PBS) with at least 50% ethanol, methanol, or isopropanol or 10mM Tris-EDTA pH8 or 4M guanidine hydrochloride. The wash buffer may be removed using any of the techniques described above for removing the solvent from the sample.

The isolated target nucleic acid may be eluted off of the membrane or retained on the membrane for further analysis. Where it is desirable to elute a target nucleic acid, an elution buffer may be used to harvest a target nucleic acid from the first membrane or the second membrane. Alternatively, the elution buffer may elute material, e.g., unwanted nucleic acids, off of the first membrane or the second membrane thereby leaving the target nucleic acid on the first membrane or the second membrane. The elution buffer may be selected according to the type of nucleic acid to be isolated. As an example, where the nucleic acid is RNA, the elution buffer may be RNAse free water, which may be used to elute the target RNA from the second membrane or from the first and second membranes when in contact or stacked on one another. Other suitable elution buffers may include tris-ethylenediaminetetraacetic acid (TE) buffer and 10mM Tris-HCl pH 8.0.

In certain embodiments where the filter device comprises more than two membranes, the target nucleic acid may be eluted off of the first or second membrane, or both the first and second membrane onto a third membrane. The third membrane may comprise a capture probe, e.g. a specific binding partner for the target nucleic acid such that the target nucleic acid is preferentially retained on the third membrane as a result of a specific chemical interaction between the target nucleic acid and the target probe. The capture probe may interact covalently or non-covalently with the target nucleic acid. The interaction may include charge-charge interactions, hydrogen bonds, hydrophobic interactions, van Der Waals forces and dipole-dipole interactions. Examples of capture probes include oligos which are complementary to a particular nucleic acid sequence of interest, as well as peptides, proteins and small molecules which bind nucleic acids. As an example an antibiotic, e.g. edeine which specifically binds 16s RNA may be bound to the membrane. Another example of a capture probe may include an oligo dT probe suitable for capturing polyA RNA, e.g. mRNA. The membrane could be derivatized with a known ligand such as avidin or streptavidin or neutravidin. A biotinylated capture probe could be added to the membrane thereby conferring specificity to target nucleic acid sequence.

In a specific embodiment the invention provides a method of isolating nucleic acids from a cellular sample, (e.g. total cellular RNA including 23s and 16s rRNA, cellular DNA, mycoplasm DNA or RNA, viral DNA or RNA), comprising contacting one or more wells of a multiwell plate with the sample, wherein one or more of the wells in the multiwell plate is comprised of a plurality of membranes, e.g. a polymer membrane such as a mixed cellulose membrane, a silica membrane or filter such as a glass fiber filter or glass fiber membrane. The sample may contact the polymer membrane first followed by the silica membrane. The first membrane contacted by the sample may have a nominal pore size which is smaller than the nominal pore size of a subsequent membrane contacted by the sample, .e.g., the second membrane. The membranes may be arranged in a stacked configuration. A vacuum may be applied to the multiwell plate and the filtrate from the sample discarded such that the cellular sample is isolated on the first membrane. The cellular sample may be contacted with a lysis buffer, e.g. a buffer comprising one or more lysis enzymes such as lysozyme. The sample may be contacted with one or more precipitating agents such guanidine salt and ethanol. The sample may be allowed to incubate for an appropriate amount of time. A vacuum may be applied to the multiwell plate and the filtrate may be discarded. One or more wash solutions, such as PBS with 50% or greater ethanol may be applied to the multiwell device in order to wash off any reagents or non-target material. A vacuum may be applied and the filtrate discarded. A second multiwell plate, i.e. a collection plate, may be provided for receiving the target nucleic acid. The second multiwell plate may be comprised of the same number of wells as the multiwell plate which receives the sample and it may be sized to fit securely, e.g., by comprising interlocking or snap together shoulders or edges, underneath the multiwell plate such that each well of the multiwell plate aligns and corresponds with a well of the second multiwell plate.

The sample may be contacted with an elution buffer, e.g. RNase free water, TE buffer, to elute the target nucleic acid from the multiwell plate into the collection plate. Alternatively, any low ionic strength solution (0-50mM) at pH 6.0-8.0 would be suitable to elute the target nucleic acid such as RNA from the device.

### Automated Systems

In certain embodiments the invention provides an automated system for isolating a nucleic acid from a sample. Thus any, or all, of the steps may be automated, including applying the sample to the filter device, lysing the sample, washing the sample, eluting the sample. The automated system may comprise a computer, e.g. a personal computer programmed to carry out each of steps of the method described herein. The invention also contemplates processing multiple samples in a multiplex format.

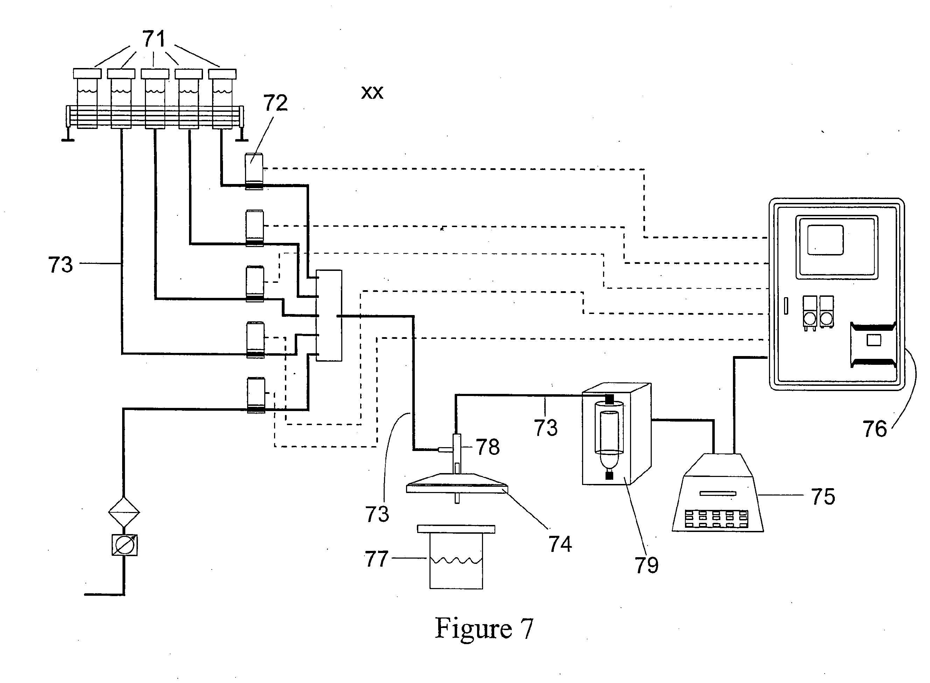

One example of an automated device is shown in Figure 7. In further embodiments the invention provides an automated system for isolating a nucleic acid from a sample comprising a) a filter device (74) suitable for isolating a nucleic acid from a sample; b) a pump (75), c) a program logic controller(PLC) (76), d) at least one container (71) suitable for holding a sample, e) optionally one or more containers (71) suitable for holding one or more reagents or buffers, f) optionally one or more receptacles suitable for receiving a filtrate from the filter device, an eluate from a filter device and/or a waste solution from a filter device (77); g) a plurality of pinch valves (72); h) tubing (73) and i) optionally a syringe cartridge positioned in a syringe barrel holder (79) which feeds one or more fluids from the tubing into the filter device (74). A check valve (78) is provided to allow application and/or removal of a fluid from the filter device.

Thus the automated system according to the invention may be configured such that fluids including reagents, buffers, samples and the like never contact any moving parts, such as the moving parts of a pump. This may be achieved by using pinch valves which require no contact with a fluid passing through the system. The tubing and the filter device used in the system may suitable for a single use and thus may be disposable. The combination of disposable tubing and pinch valves provides a system which requires little maintenance e.g, cleaning between runs. Moreover because the tubing and filter device are used only once the risk of contamination of a sample is eliminated or minimized. This is particularly useful when the sample comprises a nucleic acid and downstream detection of the nucleic acid relies on an amplification protocol such as PCR or TMA. While providing superior sensitivity, amplification protocols also entail significant risk in amplifying a contaminating nucleic acid instead the target nucleic acid. The automated system described herein minimizes or eliminates such a risk and also provides an inexpensive system for performing nucleic acid isolation when compared to more traditional chromatography systems comprised of reusable (typically, metallic) parts where the fluids moving through the system contact the moving parts of the pump.

The filter device suitable for use in the automated system may be a filter device according to any embodiment of the invention described herein. The system may be configured such that 1)the tubing provides fluid communication between the sample and the filter device; 2) the tubing provides fluid communication between the filter device and each of the one or more optional containers; 3) the pump may provide a) an external force capable of moving either a sample or a buffer or reagent from the container to the filter device and/or through the filter device and/or b)an external force capable of removing any fluid, e.g. a reagent, a buffer, a sample from a surface of a membrane, e.g. a first surface of a membrane comprising a filter device. A first surface may be a surface which contacts the fluid first, e.g. a top surface where a plurality of membranes are arranged sequentially.

In some embodiments, the PLC may control the sequence and time of steps performed by the system. Thus, the PLC may control when one or more pinch valves are opened and/or are closed. Similarly, the PLC may control the duration and timing of the application of an external force applied to one or more containers and/or the filter device. The system may also comprise a pump, such as syringe pump capable of withdrawing a set volume of a fluid from one or more containers and contacting the filter device with the fluid. The pump may be programmable such that the volume of fluid obtained from a container and/or contacted with the filter device may be specified and controlled, the rate of withdrawal and/or application of a fluid from a container and/or the filter device may be specified and controlled. In another embodiment the PLC may control both the timing and duration of steps performed by the system as well as the volume of fluid obtained from a container or the surface of the membrane, the flow rate of a fluid through the system and/or the rate of withdrawal and/or application of a fluid from a container and/or the filter device.

Where the device is automated the following steps may be performed. In the initial setup, all required solutions may be loaded onto solution containers. The containers may be controlled by pinch valves and plumbed to a two-way valve connected to a syringe. Syringe intake and delivery may be controlled by a syringe pump. The sample may be injected through a dual filter. The filtrate goes to waste; the membranes may be air dried of residue. The lysis solution may be injected through the dual filter. The filtrate goes to waste. The membranes may be air dried of residue. Wash solutions may be injected through the dual filters. The filtrate goes to waste. The membranes may be air-dried of residue. The elution solution may be injected through dual filters. The filtrate goes to a collection receptacle. The membranes may be air dried of residue. Detection of nucleic acid from filtrate may occur by gel, PCR, or TMA.

### Filter Devices

The use of a variety of filtration device formats is contemplated. The filter may be a simple manual device or it may be a part of automated system. The size and the number of filtration devices may be dictated by the nature of sample and the target analyte. In some embodiments, the filter device may be comprised of a solitary unit, e.g. a cartridge. The cartridge may comprise a housing, e.g. a hollow body for containing a plurality of membranes and one or more inlets and one or more outlets. The inlets and outlets may be sized for compatibility with a standard syringe or they may be sized to accommodate a vacuum source or a positive source of pressure. The cartridge may be further comprised of a filtrate cup for collecting flow through. The cartridge may be sized to fit in a centrifuge.

The filter device may be comprised of a column comprising a plurality of filters arranged sequentially within it such that a sample applied to the top of the column will contact each of the plurality of porous membranes within the column in a specified order. For example the first contacted membrane may have a nominal pore size less than a subsequent contacted membrane. As another example the filter device may be comprised of one or more sequentially arranged syringe cylinders which may be used to apply pressure or a vacuum to the device.

The filter device may be a multiplex e.g., comprised of multi-well plate, where at least one of the wells is comprised of a filter device comprising a plurality of membranes arranged sequentially such that a sample applied to the filter contacts a first membrane followed by one or more subsequent membranes. At least one well of the multiplex may be configured with any of filtration devices described herein, e.g., where the first membrane has a smaller nominal pore size than the subsequent membrane. The filter device may be a multiplex e.g., comprised of multi-well plate, where at least one of the wells is comprised of a filter device comprising a plurality of membranes, as described in various embodiments herein. Thus at least one well of a multiwell plate may be comprised of a plurality of membranes arranged sequentially such that a sample applied to the filter contacts a first membrane followed by one or more subsequent membranes, e.g., where the first membrane is comprised of a polymer and the second membrane is comprised of silica.

Configuring all of the wells of a multi-well plate with the filter device described above is also contemplated. Suitable multi-well plates include plates comprised of a plurality of wells, e.g. 6 wells, 12 wells, 48 wells, 96 wells, 384 wells or more. The multi-well plates may be stacked one on top of another. Additionally, filter devices of the invention comprised of a single well plate are also contemplated. In some embodiments a plate for receiving flowthrough and waste material may be provided, i.e. a drain plate. The drain plate may be comprised of a single well or a plurality of wells depending on the particular analyte and sample source and whether or not subsequent analysis of the flow through is necessary. At least one of the plates, may be comprised of one or more inlets suitable for delivering a sample or a buffer solution to the filter device. At least one of the plates may be comprised of an outlet thus facilitating removal of flow through and waste material. The outlet may be sized to accommodate a connection to a vacuum source. The multi-well plate may be sized to fit in a centrifuge to facilitate sample processing once the sample is applied to the filter device.

In one specific embodiment the filter device may be comprised of a mixed cellulose ester membrane, e.g. having a nominal pore size of 0.45 microns overlaid over an APFF glass fiber membrane (Millipore Corp, Billerica, MA). Where the filter device is configured as a cartridge the membranes may have a diameter of 13 mm. Alternatively, where the filter device is configured as a multi-well plate, the membranes may be sized to fit commercially available multiwell plates. In another specific embodiment the filter device may comprise a polymer membrane comprised of a sulfonyl group having a nominal pore size of 28 nanometers overlaid over a glass fiber membrane having a nominal pore size of 0.7 microns.

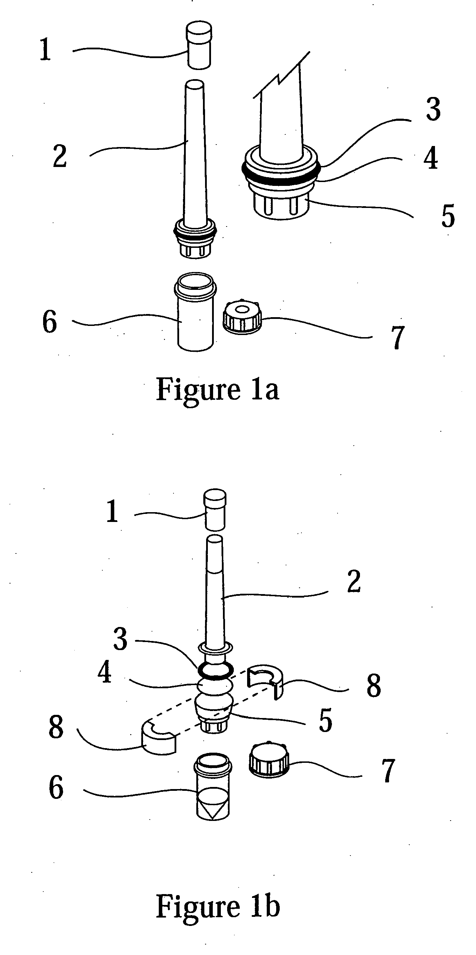

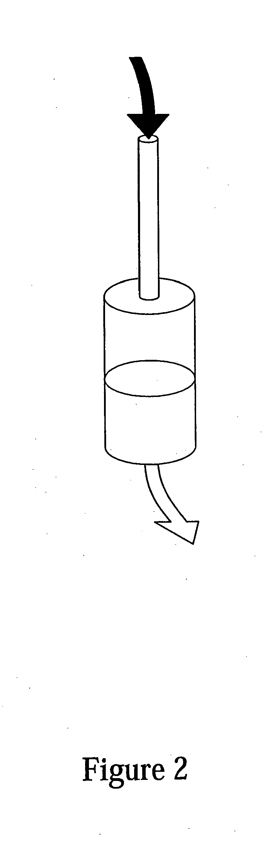

Figure 1 shows an example of a single use reusable filter device comprised of component parts according to the instant invention. The sample reservoir cap (1) is removed and the sample is applied to the sample reservoir (2) and the sample flows as a result of gravity or some external force applied to the device such that the sample contacts a first porous membrane of a plurality of porous membranes (4). The plurality of porous membranes may be seated on a membrane support (5) which is in fluid communication with a filtrate cup which may also serve as a receptacle for an eluted target. A filtrate cap (6) is also provided. Because the device may be disassembled and the plurality of porous membranes replaced, it is suitable for reuse. Figure 2 shows an example of single use filtration device of the instant invention which is not re-useable.

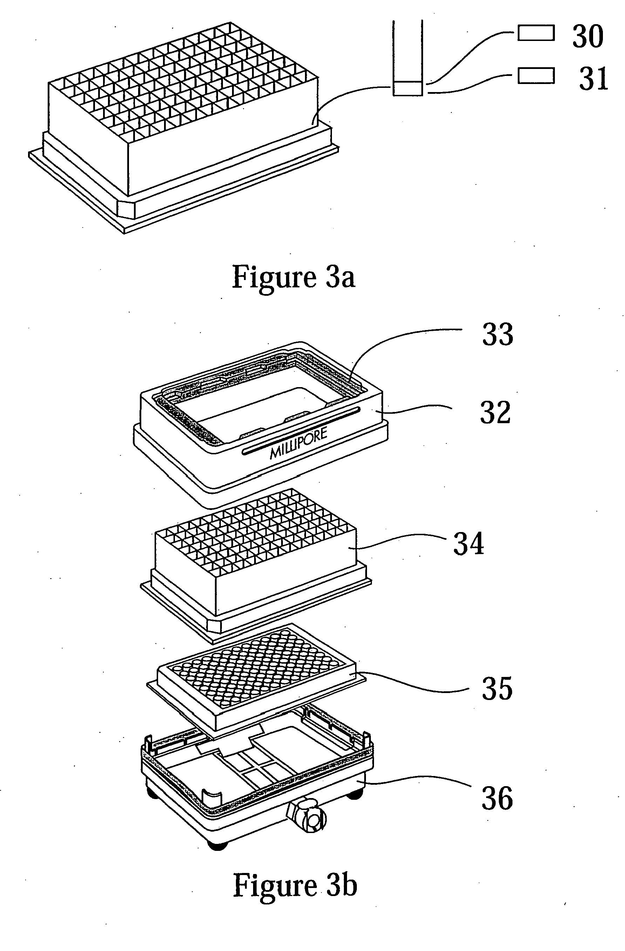

Figure 3a shows an example of a multiwell filtration device according to the present invention. Each well comprises at least two membranes (30, 31) stacked one upon the other. Figure 3b shows another example of an apparatus comprising a multiwell filtration device according to the present invention. The apparatus may include a removable collar (32), which comprises a collar gasket seated within the collar (33). The collar (32) engages a filter plate (34) comprising a plurality of wells, wherein at least one of the wells is comprised of two or more porous membranes, and wherein the first porous membrane has a smaller nominal pore size than the second porous membrane. The porous membranes may be stacked one upon the other. The filter plate (34) may be seated on a collection plate (35) which is comprised of a plurality of wells. The collection plate wells may correspond one to one with the wells of the filter plate. The collection plate may be seated on a base (36) which may comprise a vacuum manifold.

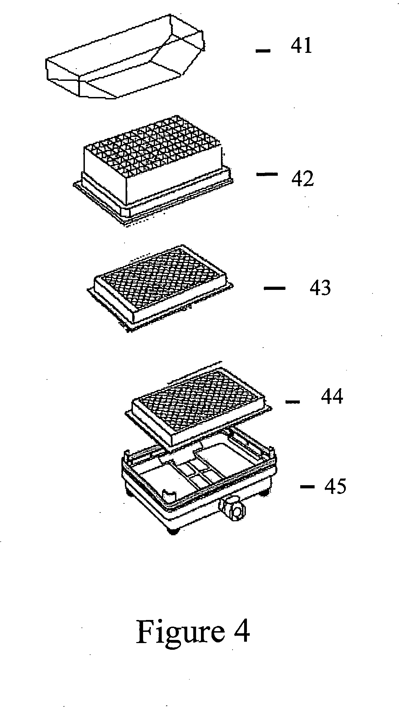

Figure 4 shows another example of a multiwell filtration device according to the present invention. The device may comprise a hopper (41) for receiving a sample and which contacts a removable filter plate comprised of a plurality of wells for receiving samples, e.g. large volume samples, where at least one of the wells is comprised of one or more porous membranes (42). The filter plate may be seated on a multiwell plate e.g., Ziptip® (Millipore Corp., Billerica, MA)(43). The wells of the Ziptip® plate may correspond one to one with the wells of the filter plate. The Ziptip® may comprise a resin or other solid support suitable for retaining a nucleic acid, such as RNA. In specific embodiments the resin may be comprised of poly A, poly U, or poly T sepharose. The Ziptip® plate may be seated on a collection plate comprised of a plurality of wells (44). The wells may correspond one to one with the wells of the Ziptip® plate and may contain TMA reagents such as reconstituted amplification reagent with amplification oligos reconstituted enzyme. The collection plate may be seated in a base comprising a multiscreen vacuum manifold (45).

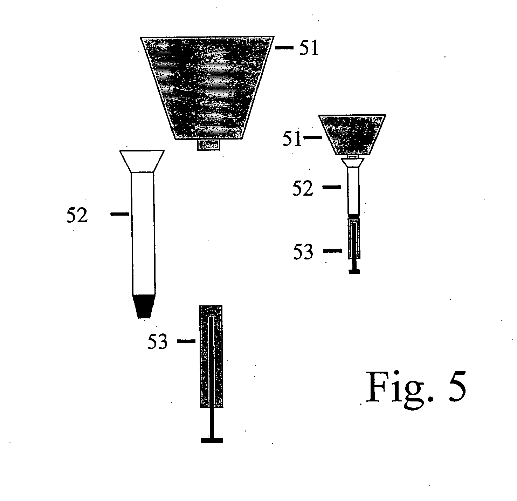

Figure 5 shows another example of a single sample filtration device according to the instant invention. The device comprises a receptacle such as Microfil® or Milliflex® (51) (Millipore Corporation, Billerica, MA) comprising one or more porous membranes suitable for retaining cells. The receptacle may be seated on a micro-column loaded with nucleic acid capture resin suitable for retaining a target nucleic acid analyte (53). The micro column may be in fluid communication with a syringe (53) suitable for pulling lysate through the column.

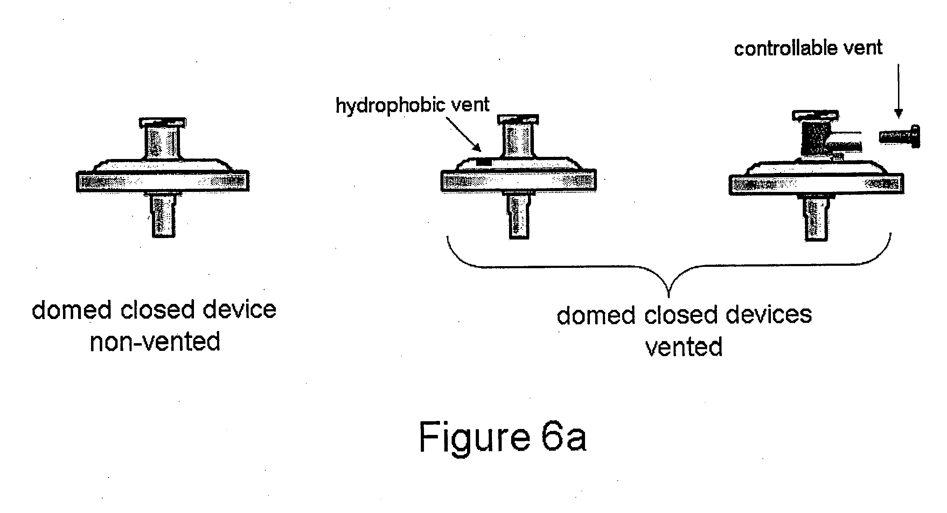

Figure 6a shows three examples of sealed closed dome filtration devices. The closed dome device may provide for processing a sample under sterile conditions. The dome may serve as housing for one or more porous membranes and may further comprise one or more vents, which do not serve as inlets for samples or reagents. The vents may be comprised of any suitable material which allows for the passage of air and other gases but prevents particulate matter such as microbes from passing. In one embodiment the vent is comprised of a gas accessible hydrophobic barrier. A hydrophillic barrier is also contemplated. In some embodiments the vent may be positioned on a surface of the domed housing structure. In another embodiment the vent may be positioned as a side arm leading into a portion of the domed housing such as an inlet in communication with the portion of the domed housing containing the one or more porous membranes. When a sample is flowing through the filter device the vent may be kept closed by using a luer-locked plug. The plug may be comprised of solid material, or may itself be vented and thus comprised of a membrane barrier such as a hydrophobic membrane. The use of vents provides a means of relieving pressure and thus preventing damage to the filter.

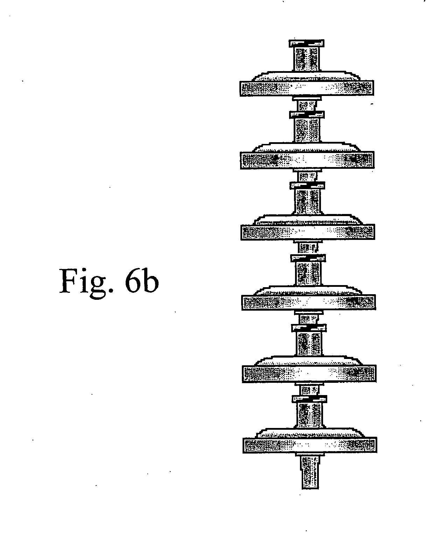

Figure 6b shows an example of a single use membrane device according to the invention where the device comprises a plurality of stacked membrane cartridges. Each cartridge in the device is in fluid communication with the next adjacent cartridge. The cartridge may be comprised of one or more porous membranes. Where the cartridge comprises more than one porous membrane, the membranes may be the same or different from the other porous membranes contained within the cartridge. In some embodiments the multiple membranes within a cartridge may be fused using heated polypropylene. As an example the first cartridge may be comprised of a mixed cellulose ester (MCE) membrane and the second cartridge stacked beneath the first may be comprised of a glass fiber membrane. The MCE may have a nominal pore size that is smaller than the glass fiber filter.

Figure 7 shows an example of a multiple membrane vented device according to one embodiment of the invention. The device may be connected by two-way vacuum valve (check valve assembly) to a syringe. The 2-way vacuum valve may be plumbed to a solution manifold. From the solution manifold emanate multiple lines connected to solution containers (i.e. sample, lysis, wash, elution solutions/buffers) and an air vent. Each solution line may be controlled by an electrical pinch valve. Sample, followed by lysis buffer, followed by washes, followed by elution buffer may each be sequentially introduced in order to capture and lyse cells on a surface of the first porous membrane in the device and then capture, wash, and elute nucleic acid from the stacked membranes in the device. The first porous membrane of the device may be comprised of a polymer such as MCE, PES. The second may be comprised of silica such as glass fiber. The nominal pore size of the polymer membrane may be smaller than the nominal pore size of the silica membrane. The system shown in Figure 7 is one example of how automation may be implemented.

### Membranes

A significant advantage of using membranes over porous beads for analyte absorption or chromatography is the difference in liquid flow patterns between the two formats. When using porous beads such as in packed beds, an analyte in an applied convective flow diffuses to the film surface and also then slowly diffuses within the pores of the bead. When using a membrane, an analyte in an applied convective flow quickly moves though a membrane and only needs to diffuse to the film surface. Saturation binding of an analyte can potentially be more quickly achieved using membranes than beads. By membrane what is meant is a structure, having lateral dimensions much greater than its longitudinal dimensions, through which mass transfer may occur under a variety of driving forces.

In addition to normal flow devices, in which bulk convection is perpendicular to the membrane surface, radial flow devices can also be used for analyte absorption and chromatography. In radial flow devices, flow is through a membrane wrapped around porous cylindrical frits. The flow can occur from inside out or outside in.

Membranes used in the invention may be comprised of any porous material known in the art. Examples of suitable porous materials, include, but are not limited to polyether sulfone, polyamide, e.g., agarose, cellulose, a polysaccharide, polytetrafluoroethylene, polysulfone, polyester, polyvinylidene fluoride, polypropylene, a fluorocarbon, e.g. poly (tetrafluoroethylene-co-perfluoro(alkyl vinyl ether)), poly carbonate, polyethylene, glass, polycarbonate, ceramic, nylon, metal and carbon based material such as graphite fiber and carbon nanotubes.

In certain specific embodiments the first membrane may be comprised of mixed cellulose, such as a mixed cellulose ester membrane (MCE) or a polymer comprised of a sulfone group such as polyethersulfone (PES). The second membrane may be comprised of glass or glass fibers. In some embodiments a resin suitable for retaining nucleic acid, e.g., RNA may be used in place of a second membrane. If an optional third membrane comprising a capture probe is desired, the third membrane may be comprised of nylon.

A skilled artisan will appreciate that other membranes comprised of material suitable for use in the invention may be chosen based on the hydrophobicity and the zeta potential of the membrane. All particulate or macroscopic materials in contact with a liquid acquire an electronic charge on their surfaces. Zeta potential is an indicator of this charge and can be used to predict and control the stability of colloidal suspensions or emulsions. The streaming potential/streaming current method may be used to determine the zeta potential of the membranes (see, e.g., Lu et al., 2006, *J. Colloid. Interface Sci.* 299(2)972; Haggard et al., 2005, *Langmuir* 21 (16):7433; Werner et al. 1998, *J. Colloid Interface Sci.* 208(1):239). Zeta potential is a measure of the surface electrostatic state of a material. If the interaction of nucleic acid with a material is predominantly guided by electrostatics then two materials having similar zeta potential profiles throughout a pH range should respond similarly in their ability to interact with nucleic acids under the same set of conditions. Nucleic acid purification protocols which involve switching from high salt to low salt conditions would be expected to be sensitive to the zeta potential of the material used to capture nucleic acid.

Membranes of the invention may have a pore size ranging from 0.01 µm to 300 µm, 0.1 µm- 100 µm, 0.1 µm - 50 µm, 0.1 µm -20 µm. In certain embodiments at least one of the membranes has a nominal pore size of 0.45 µm. In specific embodiments at least one of the membranes has a nominal pore size of 0.2 µm. The membranes of the invention may have thickness ranging from 0.1 mm to 10 mm. Thickness refers to the distance from one outer surface to another outer surface and corresponds to the distance a sample will travel as it traverses the membrane when a force, e.g. gravity, a vacuum, is applied to the membrane. The membranes may be symmetric or asymmetric. The membranes may themselves be comprised of one or more layers.

### Nucleic Acid Samples

Sample, as used herein, refers to material comprising nucleic acid derived from any biological source. The biological source may be any living thing such as a plant, an animal, a viral particle. The biological source include a whole organism or an organ or tissue derived from an organism. The biological source may be unicellular, e.g., a bacteria, a mycoplasm, or multicellular, e.g. an animal or plant, or non-cellular, e.g. viral. Cellular sources may include eukaryotic cells, prokaryotic cells. Examples of eukaryotic cells may include cells derived from any mammal, e.g., humans, non-human primates, horses, goats, sheep, rats, rabbits, mice, guinea pigs. Examples of prokaryotic cells include gram negative bacteria and gram positive bacteria such as *Escherichia coli, Pseudomonas aeruginosa, Staphylococcal aureus.* The sample may include whole cells or lysates thereof. The sample may include naturally occurring samples or those created or manipulated either partially or wholly by the human hand.

Target nucleic acids for isolation according to the methods disclosed herein include any nucleic acid found in a sample including DNA, RNA, PNA, LNA, and hybrids of more than one type of nucleic acid. The nucleic acid may be double stranded, single stranded, or multi-stranded. Where the target is DNA, the DNA may be, plasmid DNA, vector DNA, genomic DNA, including mitochodrial DNA, or a fragment of any of the preceding. Where the target is comprised of RNA the RNA may be mRNA, tRNA or rRNA e.g. 16s RNA, 23s RNA or any combination thereof. The nucleic acid may be comprised of nucleotide analogs, e.g., chain terminators. The size of the target nucleic acid may range from 2-30 nucleotides or may be in the kilobase or larger range.

### Kits

The invention also provides kits which may be used to isolate nucleic acids from a sample. The kit may comprise one or more filtration devices according to the instant invention and one or more containers. The kit may contain one or more controls or sample target nucleic acids or specimens and may optionally include various buffers useful in the methods of the invention. As an example the kit may include a lysis buffer suitable for lysing viral particles or cells. Wash buffers for eliminating reagents or non-specifically retained or bound material may optionally be included in the kit. Other optional kit reagents include an elution buffer for eluting a bound target nucleic acid from a membrane. Each of the buffers may be provided in a separate container as a solution. Alternatively the buffers may be provided in dry form or as a powder and may be made up as a solution according to the users desired application. In this case the buffers may be provided in packets. The kit may provide a power source in instances where the device is automated as well as a means of providing an external force such as a vacuum pump. The kit may also include instructions for using the device and/or for making up reagents suitable for use with the device and methods according to the instant invention. Optional software for recording and analyzing data obtained while practicing the methods of the invention or while using the device of the invention may also be included.

### Methods and Oligonucleotides for Detecting Microorganisms

In certain embodiments the invention provides oligonucleotides suitable for detecting a microorganism in a sample. The oligonucleotides may be comprised of DNA or RNA and may specifically bind to at least a portion of the genome of a microorganism. The microorganism may be a virus such as a parvovirus including minute virus of mouse (MVM) or a mycoplasma.

Bind specifically as used herein refers to nitrogenous base pairing, e.g. nucleotides comprising guanine will bind specifically to its complement i.e. a nucleotide comprising cytosine. Similarly, nucleotides comprising adenine will specifically bind to its complement i.e. a nucleotide comprising thymidine. Typically a sequence may specifically bind its complement under moderate stringency conditions.

The one or more oligonucleotides may comprise a short nucleic acid sequence such as a DNA sequence, or an RNA sequence, which binds specifically to at least a portion of a genome of one or more species of mycoplasma or MVM. Typically the oligonucleotides range from 10 nucleotides to 40 nucleotides, from 15 nucleotides to 35 nucleotides, from 20 nucleotides to 30 nucleotides in length.

The invention thus provides isolated DNA sequences selected from: (a) DNA comprising the nucleotide sequence of SEQ ID NOS: 4, 5, 6, 7, 8, 9, 10, 11, and/or 12; (b) DNA capable of hybridization to the DNA encoded by any of SEQ ID NOS: 4, 5, 6, 7, 8, 9, 10, 11, and/or 12 under conditions of moderate stringency; (c) DNA capable of hybridization to a DNA of (a) under conditions of high stringency.

In another embodiment, the nucleic acid molecules of the invention also comprise nucleotide sequences that are at least 80% identical to SEQ ID NOS: 4, 5, ,6, 7, 8, 9, 10, 11, and/or 12. Also contemplated are embodiments in which a nucleic acid molecule comprises a sequence that is at least 90% identical, at least 95% identical, at least 98% identical, at least 99% identical, or at least 99.9% identical to SEQ ID NOS: 4, 5, 6, 7, 8, 9, 10, 11, and/or 12. Percent identity may include identity over the entire length of the sequence, e.g. one or more nucleotides may be substituted with one or more different nucleotides, or it may include a truncation of a portion of the sequence or a combination of both. The percent identity may be determined by visual inspection and mathematical calculation. Alternatively, the percent identity of two nucleic acid sequences can be determined by comparing sequence information using the GAP computer program, version 6.0 described by Devereux et al. 1984, Nucl. Acids Res. 12:387 and available from the University of Wisconsin Genetics Computer Group (UWGCG). The preferred default parameters for the GAP program include: (1) a unary comparison matrix (containing a value of 1 for identities and 0 for non identities) for nucleotides, and the weighted comparison matrix of Gribskov and Burgess 1986, Nucl. Acids Res. 14:6745, as described by Schwartz and Dayhoff, eds. 1979, Atlas of Protein Sequence and Structure, National Biomedical Research Foundation, pp. 353-358 (2) a penalty of 3.0 for each gap and an additional 0.10 penalty for each symbol in each gap; and (3) no penalty for end gaps. Other programs used by one skilled in the art of sequence comparison may also be used.

Moderate stringency, as used herein, include conditions that can be readily determined by those having ordinary skill in the art based on, for example, the length of the DNA. The basic conditions are set forth by Sambrook et al. 1989, Molecular Cloning: A Laboratory Manual, 2d ed., 1:1.101-104, Cold Spring Harbor Laboratory Press, and include use of a prewashing solution comprising 5X SSC, 0.5% SDS, 1.0 mM EDTA (pH 8.0), hybridization conditions of 50% formamide, 6X SSC at 42°C (or other similar hybridization solution, such as Stark's solution, in 50% formamide at 42°C), and washing conditions of 60°C, 0.5X SSC, 0.1 % SDS.

High stringency, as used herein, includes conditions readily determined by the skilled artisan based on, for example, the length of the DNA. Generally, such conditions are defined as hybridization conditions as above, and with washing at approximately 68°C, 0.2X SSC, 0.1 % SDS. The skilled artisan will recognize that the temperature and wash solution salt concentration can be adjusted as necessary according to factors such as the length of the oligonucleotide.

Two of the oligonucleotides may serve as primers in a polymerization reaction, e.g., PCR including RT-PCR, real time PCR. In PCR two primers are used which hybridize to a specific DNA sequence thus facilitating the initiation of DNA synthesis by the polymerase. The primers bind to complementary DNA strands and initiate DNA synthesis in opposite directions. A third oligonucleotide may serve as probe for detecting the PCR amplified sequence by binding to at least a portion of a nucleic acid sequence contained within the amplified sequence. At least one of the oligonucleotides may be a peptide nucleic acid (PNA). A peptide nucleic acid is a nucleic acid molecule in which the deoxyribose or ribose sugar backbone, usually present in DNA and RNA is replaced with a peptide backbone. Methods of making PNAs are known in the art (see e.g. Nielson, 2001, Current Opinion in Biotechnology 12:16).

In certain embodiments it may be desirable to detect a target nucleic acid sequence contained in microorganism. Thus, at least one of the oligonucleotides may be modified with a detectable substance. Suitable detectable substances include a dye such as a fluorescent dye, a radioactive label such as ³²P and a molecule which is detected by binding of one or more additional molecules. For example an oligonucleotide may be modified by the addition of digoxigenin, which may be detected by binding one or more antibodies. The antibodies may be modified to include one or more detectable substances as well. The detectable substance may be placed on an oligonucleotide probe, or alternatively the detectable substance may be placed on one or more of the oligonucleotides which serve as primers in an amplification reaction.

Where the detectable substance is fluorescent dye used in real time PCR the oligonucleotide may be labeled with a quencher as well. Real time PCR is commonly carried out with a probe with a fluorescent reporter and a quencher held in adjacent positions. The close proximity of the reporter to the quencher prevents its fluorescence, it is only on the breakdown of the probe that the fluorescence is seen. This process depends on the 5' to 3' exonuclease activity of the polymerase involved. On melting of the DNA the probe is able to bind to its complementary sequence in the region of interest of the template DNA (as the primers will too). When the PCR is heated to activate the polymerase, the polymerase starts writing the complementary strand to the primed single strand DNA. As the polymerization continues it reaches the probe bound to its complementary sequence. Chemically the polymerase "overwrites" the probe, breaking it into separate nucleotides, and so separating the fluorescent reporter and the quencher. This results in an increase in fluorescence. As PCR progresses more and more of the fluorescent reporter is liberated from its quencher, resulting in a well defined geometric increase in fluorescence. This allows accurate determination of the final, and so initial, quantities of DNA.

In specific embodiments the invention provides one or more oligonucleotides suitable for detecting a mycoplasma in a sample. Surprisingly, the inventors have discovered a plurality of oligonucleotides which specifically bind to at least a portion of the genome of a plurality of mycoplasma species. Thus in certain embodiments the oligonucleotides bind specifically to at least 12 species of mycoplasma. Mycoplasma species which may be detected using one or more oligonucleotides according to the invention include *M. orale, M. hyorhinis, M. gallisepticum, M. pneumoniae, M. synoviae, M. fermentans, A. laidlwaii, M. arginini, M. hominis, M. pirum, M, salivarium, M. arthritidis.*

The invention also provides a method of detecting a microorganism in a sample comprising a) isolating a nucleic acid from the sample; b) contacting the isolated nucleic acid with a plurality of oligonucleotides according to the invention; c) amplifying at least a portion of the isolated nucleic acid from the sample thereby detecting the microorganism in the sample. The method may further comprise an optional step of contacting the amplified nucleic acid with an oligonucleotide probe that is labeled with a detectable substance. Alternatively in some embodiments, for example if real time PCR is used, step b) may include contacting isolated nucleic acid with at least one oligonucleotide comprised of a detectable substance, e.g. a probe.

### Examples

### Example 1: Isolation of rRNA from Pseudomonas aeruginosa

An overnight pseudomonas aeruginosa culture grown in 4mls of tryptic soy broth (TSB) was transferred for growth to log phase at 35°C in 20ml of TSB. The measured optical density at 600nm was 0.888; plate count revealed a colony concentration of 4.1 E08 colony forming units/milliliter (cfu/ml). The culture was diluted 100 fold in TSB (4.1 E06 cfu/ml).

A Millipore micropartition device (Figure 1) (Millipore Corporation, Billerica, MA) was fitted with a 13 mm APFF 0.7 micron glass filter (bottom membrane) and a 13mm mixed cellulose ester (MCE) 0.45 micron membrane (top membrane) in place of the provided YMT membrane. A second Millipore micropartition device was fitted with only a 13 mm MCE 0.45 micron membrane in place of the provided YMT membrane.

A positive control comprising 1 ml of 4.1 E06 cfu pseudomonas aeruginosa in TSB was centrifuged to a pellet for 10 minutes at 10,000 rpm, 4°C.

To the micropartition device was added 1ml of pseudomonas aeruginosa (4.1 E08 cfu or 4.1 E06 cfu). The loaded devices were centrifuged at 4000 rpm for 5 minutes at 4°C. The filtrate was discarded. Hen egg white lysozyme (100 microliters, 0.4mg/ml) in TE was added to the micropartition device. After 10 minutes of contact with the top membrane at room temperature, 600 microliters of a solution comprising 350ul of RLT buffer comprising 25-50% guanidinium thiocyanate from Qiagen RNAeasy Minikit® (Qiagen, Inc., Valencia, CA) and 250 microliters of ethanol were added and allowed to sit on the top membrane for 5 minutes. The devices were centrifuged at 4000 rpm for 5 minutes at 4°C. The filtrate from the micropartition device with 2 membranes was discarded. The filtrate from the micropartition device with just the top membrane was added to Qiagen mini column from the Qiagen RNAeasy Minikit® (Qiagen, Inc., Valencia, CA). The positive control pellet was subjected to lysis in a microtube in a similar way and then also added to a Qiagen RNAeasy Minikit® column (Qiagen, Inc., Valencia, CA). The micropartition device and Qiagen columns were washed with 700 microliters of RW 1 buffer comprising 2.5-10% guanidinium thiocyanate and 2.5-10% ethanol (Qiagen RNAeasy Minikit ®)(Qiagen, Inc., Valencia, CA); centrifuged at 4°C, for 5 minutes, at 4000 rpm; and then washed twice with 500 microliters of RPE buffer from the Qiagen RNAeasy Minikit® (Qiagen, Inc., Valencia, CA). All fitrates were discarded. Elution was performed with two sequential additions of 100 microliters of RNAse free water.

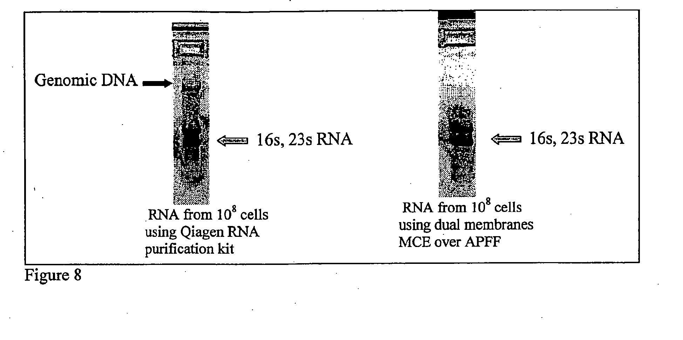

Eluant samples from the two membrane device, the one membrane device in conjunction with the Qiagen column, or just the Qiagen column alone were applied to wells of a 1.2% agarose prestained Sybersafe E-gel PowerBase™ according to the manufacturer's instructions (Invitrogen Corporation, Carlsbad, CA). The gel was allowed to run for 15 minutes. Nucleic acid bands were visualized using UV excitation and blue light emission on a LAS-3000 (Fujifilm Global, Japan). The results demonstrate rRNA isolated from the dual membrane filtration device of the invention yielded 16s and 23s RNA free from any apparent genomic DNA contamination. In contrast, the sample run through the Qiagen column had significant detectible genomic DNA band (Figure 8). The sample lysed on the MCE filter and subsequently run through the Qiagen column had no apparent genomic DNA contamination (data not shown).

### Example 2: Quantification of isolated RNA

RT-PCR was used to quantify the RNA isolated from the samples processed through the MCE membrane followed by the glass membrane as well as the MCE membrane followed by the Qiagen column (Qiagen, Inc., Valencia, CA) as described above in Example 1. The bacterial cell pellet (also described above in Example 1) served as a control.

The following 23S *Pseudomonas aeruginosa* specific primers were purchased:
PAF1-23S(TAM), forward primer, CACACGGCGGGTGCTAAC (Sigma-Aldrich, St Louis, MO) (SEQ ID NO: 1)
PAR1-23S(TAM), reverse primer, CCACAACTTTGGGACCTTAGCT (Sigma-Aldrich, St Louis, MO) (SEQ ID NO: 2)
PAP1-23S(TAM), FAM labeled probe, CCGTCGTGAAAAGGGAAACAACCCA (Applied Biosystems, Inc., Foster City, CA) (SEQ ID NO: 3).

Real time PCR was performed using the probe above labeled with a fluorescent tag and a quencher. Production of the PCR product resulted in the cleavage of the tag and quencher on the probe and thus resulted in the production of a fluorescent signal that was used in conjunction with a standard curve to quantify the PCR product. *Pseudomonas* aeruginosa rRNA transcript of known concentration was used to generate a standard of emergence time (CT) and nucleic acid molecules. The threshold cycle (CT) refers to the fractional cycle number at which the fluorescent signals for the reactions of the RT-PCR cross a pre-defined fluorescent threshold. In order to correlate rRNA copy number to CT value, a linear relationship between a known amount of pseudomonas aeruginosa rRNA and CT value was first established. This standard curve was then used to determine the copy number of pseudomonas aeruginosa rRNA purified from the processed samples.

A one-step RT-PCR master mix kit (ABI) was used. To a final volume of 15 microliters, forward primer (300nM) was mixed with reverse primer (10nM), probe (50nM), 2X RT-PCR mix, reverse transcriptase, and 10 microliters of eluant RNA from samples. In an ABI 7000 cycler (Applied Biosystems, Inc., Foster City, CA), the following cycling program was executed:
1. 50°C, 30:00 minutes
2. 95°C, 10:00 minutes
3. 95°C, 0:15 minutes, 40 cycles
4. 62°C, 1:00 minutes

Quantification of the RNA present in the samples and the positive and negative controls (no template) with and without reverse transcriptase was performed in triplicate.

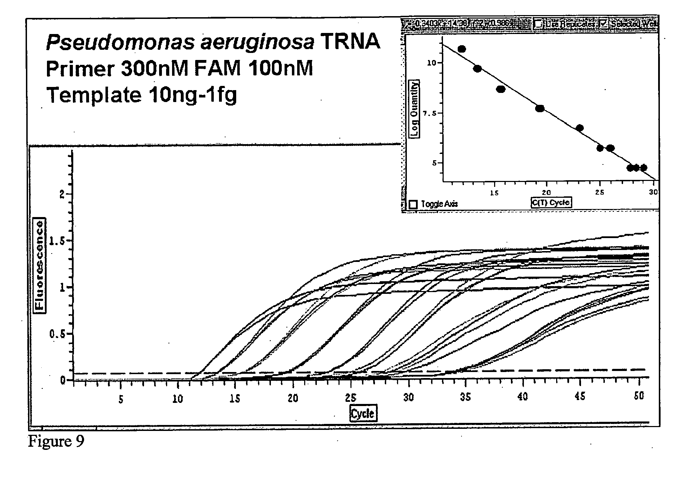

The extent of selective RNA purification obtained by using a MCE membrane prior to capture on a glass filter was demonstrated using quantitative reverse transcriptase polymerase chain reaction (qRT-PCR)(real time PCR), Figure 9 and Table 1. The relative quantity of DNA to RNA for each sample was established by performing PCR in the presence and absence of reverse transcriptase for each sample. In the absence of reverse transcriptase only the genomic DNA was amplified. In the presence of reverse transcriptase both DNA and RNA were amplified when PCR was subsequently performed, but the starting copy number of RNA for the particular target sequence was substantially in excess (by ≥ a factor of 1000) relative to the starting copies derived from genomic DNA. Because of this fact, when reverse transcriptase was used with PCR for ribosomal RNA, the amplified signal observed was essentially just that of the RNA. By determining the ratio of the target gene copy number (derived from a standard curve with known starting nucleic acid concentrations) in the presence and absence of reverse transcriptase, a dramatic reduction in genomic contaminating DNA was achieved with lysis of captured cells on the MCE membrane. The copy number for the amplified nucleic acid signal was determined by correlating the experimental CT value to its corresponding copy number value on a standard curve defined with a known amount of pseudomonas aeruginosa rRNA. The noRT (no reverse transcriptase) copy numbers represent the number of DNA copies (or the level of DNA contamination if RNA is desired) present in the sample. If the pellet control was set as a standard, then the extent of RNA purification achieved by the use of a MCE membrane or a dual membrane approach was readily determined (Table 1). The copy numbers for the noRT reactions of the three samples had the following values: positive control (6.63E+07 copies), MCE membrane then Qiagen column (1.42E+06 copies), MCE/APFF membrane (3.05E+05 copies). Samples which are processed through MCE membrane exhibit far less recoverable DNA and thus provide for a purer RNA sample. Thus a relative purification approaching two orders of magnitude was achieved using a MCE membrane (Figure 9).

**Table 1**

| **Sample** | **Condition** | **Ct.1** | **Ct.2** | **Ct.3** | **Average** | **Stdev** | **Copies** | **Copies** | **Copies** | **Average** | **Relative Fraction genomic DNA to pellet and QIagen kit** | **percent reduction of genomic DNA relative to control pellet** |
|---|---|---|---|---|---|---|---|---|---|---|---|---|
| **4.1E06 Pellet then Qiagen kit (positive control** | RT | 12.15 | 12.16 | 12.11 | 12.14 | 0.03 | 1.78E+10 | 1.77E+10 | 1.83E+10 | 1.79E+10 | | |
| | NoRT | 19.25 | 19.33 | 19.20 | 19.29 | 0.04 | 6.81E+07 | 6.40E+07 | 6.69E+07 | 6.63E+07 | 1.00E+00 | 0.0 |
| | | | | | | | | | | | | |
| **4.1E06 MCE membrane then Qiagen column** | RT | 12.18 | 12.25 | 12.09 | 12.17 | 0.08 | 1.74E+10 | 1.64E+10 | 1.87E+10 | 1.75E+10 | | |
| | NoRT | 24.28 | 24.1 | 24.18 | 24.19 | 0.10 | 1.31E+06 | 1.52E+06 | 1.43E+06 | 1.42E+06 | 2.14E-02 | 97.9 |
| | | | | | | | | | | | | |
| **4.1E06 MCE/APFF membrane** | RT | 13.74 | 13.61 | 13.45 | 13.60 | 0.15 | 5.09E+09 | 5.64E+09 | 6.42E+09 | 5.72E+09 | | |
| | NoRT | 26.22 | 26.22 | 26.02 | 26.15 | 0.12 | 2.88E+05 | 2.88E+05 | 3.39E+05 | 3.05E+05 | 4.60E-03 | 99.5 |
| | | | | | | | | | | | | |
| **NTC (no template control)** | RT | N/A | 29.76 | N/A | 29.76 | N/A | ND | 1.80E+04 | ND | 1.80E+04 | 2.72E.04 | 100.0 |

### Example 3: Multiscreen Analysis of isolated RNA

*Pseudomonas* aeruginosa cells (1.83E06 cfu) were captured on a 0.01 % peptone prewetted 0.45 micron MCE 96 well plate (Millipore Corporation, Billerica MA) inserted on a Millipore Multiscreen manifold (Millipore Corporation, Billerica MA). The captured cells were then washed with 300ul of 0.01% peptone. A 1.0 micron glass fiber type B plate (Millipore Corporation, Billerica MA) was stacked below the MCE plate (Millipore Corporation, Billerica MA). 200 microliters of a solution comprising 100µl of RLT buffer from Qiagen RNAeasy Minikit® (Qiagen Inc., Valencia, CA) and 1000 microliters of ethanol were added and allowed to sit in the wells of the top plate for 5 minutes. After filtration of the lysate solution, the top filter plate was removed and discarded. The glass fiber filter plate was washed once with 200 microliters of RW1 buffer (Qiagen, Inc. Valencia, CA) and 200 microliters of RPE buffer (Qiagen, Inc., Valencia, CA). The filter plate was then washed twice with 300 microliters of ethanol in order to dry out the wells. A recovery collection plate (Millipore Corporation, Billerica, MA) was added below the glass filter plate. Nucleic acid was eluted from the glass fiber plate with sequential elution each of 100 microliters RNAse-free water.

To determine the relative purity of the bacterial ribosomal RNA a 48 well 2% clear agarose E-gel (Invitrogen Corporation, Carlsbad, CA) was loaded first with 5 microliters of 1:1000 diluted Syber Gold stain (Invitrogen Corporation, Carlsbad, CA) and 15 microliters of sample eluant or 60ng of 0.5-10Kb RNA ladder (Invitrogen Corporation, Carlsbad, CA). The gel was run for 30 minutes. Nucleic acid bands were visualized using UV excitation and blue light emission on a LAS-3000 (Fujifilm Global, Japan).

The entire process of cell capture, lysis, and selective nucleic acid isolation was performed using multiple filter plate types on the Millipore multiscreen manifold (Millipore Corporation, Billerica, MA). Cells were first captured by vacuum filtration on a MCE filter plate. A glass fiber filter plate was then placed underneath the MCE filter plate. Cell lysis was performed with a 50% solution of Qiagen guanidine based RLT buffer (Qiagen, Inc. Valencia, CA) and ethanol. Filtration through the two plates resulted in RNA retention on the glass fiber plate. After discarding the top MCE plate, the glass fiber plate was washed with Qiagen RW1 and RPE buffers (Qiagen, Inc. Valencia, CA). The plate was then dried by ethanol filtration. Two sequential elutions with 100 microliters of water were performed.

The results demonstrated that ribosomal RNA (16S, 23S) with no apparent contaminating genomic DNA was obtained in a multiplex format.

### Example 4: Isolation and detection of viral DNA from cell culture supernatant

Supernatant was collected from indicator cell line human NB-324K infected with the parvovirus minute virus of mouse . The viral titer of the supernatant was calculated at 8 log₁₀ TCID₅₀/ml.

Two layers of an ultrafiltration polyethersulfone membrane (nominal pore size 28 nanometer) were inserted into a sealed vented domed Millipore device (Figure 6a). A second sealed vented domed Millipore device contained Millipore APFF glass fiber membrane (0.7 micron). The devices were stacked one on top of another by luer slip to luer lock outlet to inlet connection.

A half of a ml of virus-containing cell culture supernatant was applied to the first membrane device. The second device was placed in series after the first device. 1 ml of a solution comprising 500ul of RLT buffer comprising 25-50% guanidinium thiocyanate from Qiagen RNeasy Minikit® (Qiagen, Inc., Valencia, CA) and 500 microliters of ethanol was added by syringe and allowed to sit on the top membrane for 10 minutes. Excess liquid was removed by flushing the device with air using a syringe. The in-series sealed vented domed devices were washed by syringe addition with 1ml of RW1 buffer comprising 2.5-10% guanidinium thiocyanate and 2.5-10% ethanol (Qiagen RNeasy Minikit ®)(Qiagen, Inc., Valencia, CA); and then washed twice by syringe addition of 1ml of RPE buffer from the Qiagen RNeasy Minikit® (Qiagen, Inc., Valencia, CA). All fitrates were discarded. The micropartition devices were dried by syringe addition of air. The devices were separated and elution from each was performed with addition of 500 microliters of nuclease free water.

Real time PCR was used to quantify the DNA isolated by micropartition device and Qiagen buffers. Previously quantified viral nucleic acid was used as control. Forward and reverse primers, a probe with an attached fluorescent tag and quencher, water, template and ABI 2X Universal TaqMan PCR Master Mix were added to a final volume of 20 ul. The following probes and primers specific to MVM were used;
Forward primer(MVMJ02275f1):
   GCAAACAGCATGGTGAAAATTG (SEQ ID NO: 4)
Reverse Primer (MVMJ02275r):
   CCTGAACCAAAGCTTGTTTCATC (SEQ ID NO: 5)
Probe (MVMJ02275probe1)
   TGGACCAGCACCAGAGCGCTACAC (SEQ ID NO: 6)

Cycling was performed on a BioRad Chromo4 instrument: 1) 50°C, 2:00 minutes, 2) 95°C, 10:00 minutes, 3) 95°C, 0:15 minutes, 4) 60°C, 1:00 minutes, 5) goto step 3 39 times.

**Table 2:**

| Sample | Ct1 | Ct2 | Ct3 | Average | Stdev | cv |
|---|---|---|---|---|---|---|
| 8 log₁₀ TCID₅₀/ml VR | 28.84 | 28.88 | | 28.86 | 0.03 | 0.001 |
| 8 log₁₀ TCID₅₀/ml APFF | 17.54 | 17.57 | | 17.56 | 0.02 | 0.001 |
| 8 log₁₀ TCID₅₀/ml VR | 30.24 | 30.37 | | 30.31 | 0.09 | 0.003 |
| 8 log₁₀ TCID₅₀/ml APFF | 18.57 | 20.00 | | 19.29 | 1.01 | 0.052 |
| No template control | ND | ND | ND | ND | | |
| 10^6 copies/ul (internal PCR control) | 19.65 | 19.97 | | 19.81 | 0.23 | 0.011 |

Control standard nucleic acid amplified as expected. Viral DNA was successfully recovered and detected from the APFF and the ultrafiltartion poly ethersulfone (UF PES) membranes (denoted VR in Table 2). The APFF membrane retained more nucleic acid than the UF PES membrane.

### Example 5: Isolation and quantification of rRNA from Mycoplasma hyorhinis

A one-day culture of *Mycoplasma hyorhinis* was grown in 50ml of serum containing mycoplasma broth media at 37°C and 7% CO₂. This culture had a colony concentration of 2.4 x 10⁸ cfu/ml. The culture was diluted 1000-fold in 0.1 % peptone. A culture of Chinese hamster ovary (CHO) cells was counted, and found to have a concentration of 1.1 x 10⁷ cells/ml.

A sealed domed vented Millipore device containing a 25mm Millipore polypropylene prefilter with a 10 micron nominal pore size (Millipore Corporation, Billerica, MA) with 0.5-µm over 0.1-µm polyethersulfone membrane (PP10-HVEP) was prepared. A second device containing a 25mm APFF glass filter was also prepared.

The positive control was 2.4x10⁵ M. hyorhinis in 1 ml of 0.1% peptone processed through a Qiagen RNAeasy Kit (Qiagen, Valencia, CA) according to the manufacturers suggested protocol for a solution sample containing bacteria.

Prior to sample introduction, 3ml of 0.1 % peptone were pushed through each device using a 10ml syringe. To two PP10-HVEP devices 3ml of 0.1 % peptone was added; to two PP10-HVEP devices 100µl of 100-fold-diluted *Mycoplasma hyorhinis* in 3ml of 0.1% peptone was added; to two additional PP10-HVEP devices 100µl of 100-fold-diluted *Mycoplasma hyorhinis* in 3ml of CHO cell suspension was added. The samples were added and pushed through the devices with 10ml syringes. One ml of Qiagen Buffer RLT + Ethanol (1: 1) solution was taken up into a 10ml syringe per device, and added to the device until a drop or two of fluid emerged from the bottom of the device. An APFF device was added securely to the bottom of each PP10-HVEP device. The RLT-Ethanol solution was allowed to sit on the filters for five minutes at room temperature, after which the solution was purged from the devices. The devices were separated and the device with the APFF filter was processed further. One ml of Qiagen Buffer RW1 was pushed through the APFF filter device with a 10ml syringe. One ml of Qiagen Buffer RPE was pushed through the APFF filter with a 10ml syringe, then repeated once. Five hundred microliters of nuclease-free water was then added to the APFF filter devices, pushed through, and collected in a tube.

Samples that were processed via the devices were frozen for thirty minutes in an ultrafreezer, then placed into a Savant DNA120 SpeedVac (system on Medium, Manual Setting)(Thermo Electron Corp., Waltham, MA). No Heat was applied to the samples during concentration in the SpeedVac. Dried material was resuspended in 50µl of nuclease-free water.

Real time PCR was performed. RNA transcripts of known concentration were used to generate a standard of emergence time (CT) and nucleic acid molecules. The threshold cycle (CT) refers to the fractional cycle number at which the fluorescent signals for the reactions of the RT-PCR cross a pre-defined fluorescent threshold. In order to correlate rRNA copy number to CT value, a linear relationship between a known amount of mycoplasma hyorhinis rRNA and CT value was first established. This standard curve was then used to determine the copy number of mycoplasma hyorhinis rRNA purified from the processed samples.

A one-step RT-PCR master mix kit (Applied Biosystems, Foster City, CA)) was used.
To a final volume of 25 microliters, forward primer (400 nM) was mixed with reverse primer (400 nM), probe (300 nM)(labeled with a fluorescent tag and a quencher), 2X RT-PCR mix, reverse transcriptase, and 5 microliters of eluant RNA from samples.
The primers and probes used in this assay were as follows:
Sequences group 1:
   Forward primer: AAATGATCCGCCTGAGTAGTATGC (SEQ ID NO: 7)
   Reverse primer: CATGCTCCACCGCTTGTGC (SEQ ID NO: 8)
   Probe: CGCAAGAGTGAAACTTAAAGGAATTGACGG (SEQ ID NO: 9)
Sequences group 2:
   Forward primer: AAACATCCCGCCTGGGTAGTACAT (SEQ ID NO: 10)
   Reverse primer: CAACATGCTCCACCACTTGTG (SEQ ID NO: 11)
   Probe: CGCAAGAATGAAACTTAAACGGAATTGACG (SEQ ID NO: 12)
In an ABI 7000 cycler, the following cycling program was executed:
   50°C, 30:00 minutes
   95°C, 10:00 minutes
   95°C, 0:15 minutes, 40 cycles
   55°C, 1:00 minutes

Quantification of RNA present in samples and positive and negative controls (no template) with and without reverse transcriptase was performed in triplicate. Results are shown in Table 3

**Table 3**

| Sample Name | CT | Std. Dev. CT | Quantity | Mean Quantity | Std. Dev. Quantity |
|---|---|---|---|---|---|
| Neg #1 | | | | | |
| | | | | | |
| | | | | | |
| Neg #2 | | | | | |
| | | | | | |
| | | | | | |
| Myco #3 | 26.27 | 0.278 | 6.95E+06 | 7.25E+06 | 1.41 E+06 |
| | 26.48 | | 6.02E+06 | | |
| | 25.93 | | 8.78E+06 | | |
| Myco #4 | 26.33 | 1.016 | 6.69E+06 | 1.25E+07 | 9.46E+06 |
| | 24.51 | | 2.34E+07 | | |
| | 26.2 | | 7.31 E+06 | | |
| CHO + Myco | 24.6 | 0.233 | 2.19E+07 | 2.01 E+07 | 3.06E+06 |
| #5 | 25.01 | | 1.65E+07 | | |
| | 24.61 | | 2.17E+07 | | |
| CHO + Myco #6 | 24.62 | 0.346 | 2.16E+07 | 2.70E+07 | 6.63E+06 |
| | 24.41 | | 2.49E+07 | | |
| | 23.94 | | 3.44E+07 | | |
| Myco Tube #1 | 24.48 | 0.287 | 2.38E+07 | 1.92E+07 | 3.99E+06 |
| | 24.97 | | 1.70E+07 | | |
| | 24.98 | | 1.69E+07 | | |
| Myco Tube #2 | 25.72 | 0.547 | 1.01 E+07 | 1.37E+07 | 5.52E+06 |
| | 24.73 | | 2.00E+07 | | |
| | 25.63 | | 1.08E+07 | | |
| Lysis Filtrate #1 | | | | | |
| | | | | | |
| | | | | | |
| Lysis Filtrate #2 | | | | | |
| | | | | | |
| | | | | | |
| Lysis Filtrate #3 | 37.79 | -1 | 2547.83 | | |
| | | | | | |
| | | | | | |
| Lysis Filtrate #4 | 39.2 | 1.376 | 963.6 | 2937.72 | 2783.902 |
| | 38.35 | | 1727.67 | | |
| | 36.51 | | 6121.88 | | |
| Lysis Filtrate #5 | 31.13 | 0.253 | 246485.42 | 223580.92 | 36721.332 |
| | 31.58 | | 181225.69 | | |
| | 31.15 | | 243031.64 | | |
| Lysis Filtrate #6 | 30.51 | 0.547 | 377207.84 | 558744.03 | 214071.602 |
| | 29.43 | | 794805.31 | | |
| | 30.09 | | 504218.94 | | |
| No RT | 35.6 | 3.695 | 11470.41 | 626558.41 | 546658.382 |
| | 29.01 | | 1.06E+06 | | |
| | 29.4 | | 811279.56 | | |

Samples Neg #1 and Neg #2 are negative controls, mycoplasma was not present. Samples Myco #3 and Myco #4 have mycoplasma but no CHO cells and were processed through membranes. Samples CHO + Myco #5 and CHO + Myco #6 have both mycoplasma and CHO cells and were processed through membranes. Sample Myco Tube #1 and Myco Tube #2 have mycoplasma pelleted and processed in tubes. Sammple lysis filtrate # 1, lysis filtrate #2, lysis filtrate #3, lysis filtrate #4, lysis filtrate # 5, and lysis filtrate #6 refer to the filtrates following addition of lysis buffer to samples Neg #1, Neg #2, Myco #3, Myco #4, CHO+ Myco #5, and CHO+Myco #6 respectively. The filtrates were processed using a Qiagen RNAeasy® Minikit Column (Qiagen, Valencia, CA) according to the manufacturer's instructions. Sample NoRT refers to amplification reactions performed in the absence of reverse transcriptase for samples Myco #3 and Myco #4 and are included as an indication of DNA contamination in the sample.

All numbers expressing quantities of ingredients, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should be construed in light of the number of significant digits and ordinary rounding approaches.

Many modifications and variations of this invention can be made without departing from its spirit and scope, as will be apparent to those skilled in the art. The specific embodiments described herein are offered by way of example only and are not meant to be limiting in any way. It is intended that the specification and examples be considered as exemplary only, with a true scope and spirit of the invention being indicated by the following claims.

## Claims

1. A method of isolating a nucleic acid from a sample comprising a) contacting a filter device comprising a plurality of porous membranes with the sample such that the sample contacts the plurality of porous membranes sequentially beginning with a first porous membrane and followed by at least a second porous membrane, b) lysing the sample on the first porous membrane, wherein the first porous membrane has a nominal pore size which is smaller than the nominal pore size of the second porous membrane c) applying an external force to the filter device, thereby isolating the nucleic acid on at least one of the plurality of porous membranes.

2. The method of claim 1, further comprising one or more of:
a) contacting the filter device with one or more buffers or wash reagents; and/or
b) contacting the filter device with an elution buffer; and/or
c) contacting the sample with a third porous membrane, and in which case optionally, wherein the third porous membrane may comprise a capture probe.

3. The method of claim 1 or 2, wherein the first porous membrane is comprised of a polymer, and in which case optionally, wherein the polymer is chosen from a polysaccharide and polyethersulfone.

4. The method of any one of claims 1 to 3, wherein the second membrane is comprised of silica, and in which case optionally, wherein the silica is a glass fiber.

5. The method of any one of claims 1 to 4, wherein the sample is comprised of:
a) cells; and/or
b) viral particles; and/or
c) mycoplasma.

6. The method according to any one of the preceding claims, wherein the nucleic acid is DNA or RNA.

7. A filter device suitable for isolating nucleic acids from a sample comprising a plurality of porous membranes arranged sequentially such that a sample applied to the filter device contacts a first porous membrane comprising either a polysaccharide or a polymer comprised of a sulfone group, followed by at least a second porous membrane comprising silica.

8. The filter device of claim 7, wherein the polysaccharide is cellulose, and in which case optionally, wherein the cellulose is a mixed cellulose ester; or the polymer comprised of a sulfone group is polyethersulfone.

9. The filter device of claim 7 or claim 8, wherein the silica is a glass fiber.

10. The filter device of any one of claims 7 to 9, further comprising a third porous membrane comprised of a capture probe.

11. The filter device of any one of claims 7 to 10, wherein the first membrane has a nominal pore size that is smaller than the nominal pore size of the second membrane.
